# EUROPEAN PATENT SPECIFICATION

(11) **EP 2 181 722 B1**
(45) Date of publication and mention of the grant of the patent: **14.01.2015**
(21) Application number: 09252528.6
(22) Date of filing: 30.10.2009
(51) Int. Cl.: A61L 24/08, A61L 26/00, A61L 31/04, A61L 31/16, A61L 31/14, A61L 27/50, A61L 27/20

(54) **Delayed gelation compositions and methods of use**
Zusammensetzungen mit verzögerter Gelierung und Verwendungsverfahren
Compositions à gélification différée et procédés d'utilisation

(30) Priority: 31.10.2008 US 109995 P; 22.10.2009 US 603806
(43) Date of publication of application: 05.05.2010
(73) Proprietor: Covidien LP, Mansfield, MA 02048 (US)
(72) Inventor: Abuzaina, Ferass, Shelton CT 06484 (US); Hadba, Ahmad, Middlefield CT 06455 (US)
(74) Representative: Soames, Candida Jane

(56) References cited:
- WO-A1-99/15211
- AU-A1- 2005 211 584
- MI F L ET AL: "Synthesis and characterization of biodegradable TPP/genipin co-crosslinked chitosan gel beads", POLYMER, ELSEVIER SCIENCE PUBLISHERS B.V, GB, vol. 44, no. 21, 1 October 2003 (2003-10-01), pages 6521-6530, XP027124796, ISSN: 0032-3861 [retrieved on 2003-10-01]

## Description

### TECHNICAL FIELD

The present disclosure provides compositions suitable for use as surgical implants and methods for making same. Implants including compositions of the present disclosure may form in vivo over an extended period of time, thereby allowing their manipulation prior to final formation.

### DESCRIPTION OF THE RELATED ART

The use of biological implants is known. However, many implants, once placed in the body, are of a pre-formed shape or rapidly form in vivo, making it difficult to adjust the size and/or shape of the implant once placed in vivo. AU 2005211584 discloses a gel composition comprising alginate calcium cations and a thereapeutic agent. Mi F L et Al: "Synthesis and characterization of biodegradable TTP/genipin co-crosslinked chitosan gel beads" POLYMER ELSEVIER SCIENCE PUBLISHERS B.V.vol 44, no. 21, pages 6521-6530 discloses a system in which Chitosan is mixed with acetic acid TTP/genipin is added to the solution

Improved implants remain desirable. Additionally, it would be advantageous to provide synthetic compositions that would be slowly resorbed by tissue, or not resorbed at all.

### SUMMARY

The present disclosure provides compositions as defined by Claim 1 which exhibit delayed gelation. The compositions include at least one polysaccharide, at least one salt, and at least one reactive component. The composition of the present disclosure includes at least one polysaccharide, optionally in solution, at least one salt, optionally in solution, and
at least one reactive component, optionally in solution, wherein at least one of the polysaccharide, salt and reactive component are in a solution, and wherein the composition forms a gel from about 3 hours to about 5 hours after the at least one polysaccharide, at least one salt, and at least one reactive component have been combined.

The composition of the present disclosure includes at least one polysaccharide, sodium alginate, at least one salt, calcium carbonate, and at least one reactive component, genipin, wherein the pH of the composition is not below 5.

Methods for forming compositions of the present disclosure, as well as methods for their use, are also provided herein. A method not forming part of the present invention includes contacting tissue with at least one polysaccharide, at least one salt, and at least one reactive component, and allowing the at least one polysaccharide, at least one salt, and at least one reactive component to form a gel, wherein the gel forms from about 3 hours to about 5 hours after the at least one polysaccharide, at least one salt, and at least one reactive component have been combined.

In embodiments, the composition of the present disclosure is for use as part of a medical procedure such as sphincter augmentation, adhesion prevention, cosmetic reconstruction, dermal filling, combinations thereof, and the like.

### BRIEF DESCRIPTION OF THE DRAWINGS

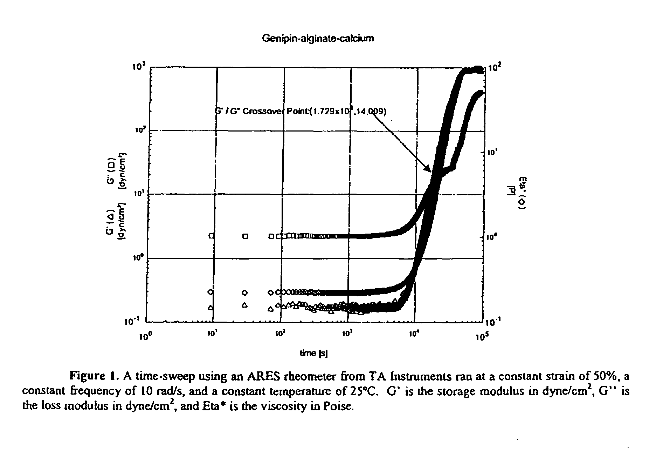

Various embodiments of the present disclosure will be described herein below with reference to the figure wherein:

The Figure is a graph showing the viscosity (Eta*, in Poise), storage modulus (G', in dyne/cm²), loss modulus (G", in dyne/cm²), and crossover point (G'/G") for a composition of the present disclosure.

### DETAILED DESCRIPTION

The present disclosure provides compositions suitable for use in forming bulking agents, adhesives, sealants, and other active implants in vivo. The compositions of the present disclosure exhibit delayed gelation. As used herein, "delayed gelation" means the compositions of the present disclosure are initially of low viscosity, i.e., the components utilized to form the compositions of the present disclosure do not begin to form a gel until from about 3 hours to about 5 hours after combining the components, in embodiments from about 4 hours to about 4.5 hours after combining the components.

Compositions of the present disclosure include at least one polysaccharide, at least one salt, and at least one reactive component. Suitable polysaccharides which may be utilized in forming compositions include, for example, starches and polysugars, including polymers containing glucosamine residues. Specific examples include, but are not limited to, carboxymethylcellulose, chitosan, pectin, alginates, glycosaminoglycans, ionizable agar, carrageen, hyaluronan, cellulose, heparin, sulfates such as chitosan sulfate, chondroitin sulfate and/or dermatan sulfate, and combinations thereof. An alginate is utilized in forming a composition of the present disclosure. Suitable alginates include, but are not limited to, sodium alginate, calcium alginate, potassium alginate, sodium alginate modified with small amounts of calcium or magnesium ions, combinations thereof, and the like. The molecular weight of the alginate may be from about 50,000 Daltons to about 500,000 Daltons, from about 60,000 Daltons to about 100,000 Daltons.

In embodiments, the polysaccharide, such as an alginate, may be in a solution. Suitable solvents for forming such a solution include any pharmaceutically acceptable solvents including, but not limited to, saline, water, alcohol, acetone, dimethyl sulfoxide, ethyl acetate, N-methylpyrrolidone, and combinations thereof. Methods for forming such solutions are within the purview of those skilled in the art and include, but are not limited to, mixing, blending, sonication, heating, combinations thereof, and the like.

The polysaccharide may be present in such a solution in an amount from about 0.1% by weight of the solution to about 10% by weight of the solution, in embodiments from about 0.5% by weight of the solution to about 5% by weight of the solution, in some embodiments about 1 % by weight of the solution.

Suitable salts for forming a composition include, for example, carbonates, bicarbonates, sulfites, phosphates, sulfates, combinations thereof, and the like. A salt such as calcium carbonate, sodium carbonate, zinc carbonate, barium sulfate, calcium phosphates, combinations thereof, and the like, may be utilized to form a composition.

In embodiments the salt may be combined with the solution possessing the polysaccharide described above. In other embodiments, the salt may be in a separate solution utilizing a solvent such as saline, water, alcohol, acetone, dimethyl sulfoxide, N-methylpyrrolidone, and combinations thereof. Methods for forming such solutions are within the purview of those skilled in the art and include, but are not limited to, mixing, blending, sonication, heating, combinations thereof, and the like.

The salt may be present in such a solution in an amount from about 0.1% by weight of the solution to about 10% by weight of the solution, in embodiments from about 0.3% by weight of the solution to about 5% by weight of the solution, in some embodiments about 0.5% by weight of the solution.

The salt solution may then be combined with the polysaccharide, optionally in solution as described above.

A composition of the present disclosure also includes a reactive component. Suitable reactive components may include crosslinkers, adhesives, sealants, couplers, and the like that are functionalized with at least one free reactive group capable of forming the composition of the present disclosure and/or linking same to tissue. More specifically, reactive components include, but are not limited to, isocyanates, N-hydroxy succinimide ("NHS"), cyanoacrylates, aldehydes (e.g., formaldehydes, glutaraldehydes, glyceraldehydes, and dialdehydes), genipin, and other compounds possessing chemistries having some affinity for the other components of the composition, tissue, or both. Reactive components may also include any natural or synthetic crosslinkers, including, but not limited to, aldehydes such as those listed above; diimides; diisocyantes; cyanamide; carbodiimides; dimethyl adipimidate; starches; and combinations thereof. The reactive components may be monofunctional, difunctional or multi-functional monomers, dimers, small molecules, or oligomers formed prior to or during implantation.

The reactive component is genipin, as shown in Structure I. 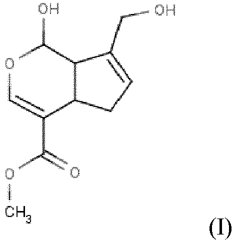 As used herein the term "genipin" includes, genipin, its derivatives, analogs, and any stereoisomer or mixture of stereoisomers of genipin. Genipin may be used as a natural crosslinker for amine-containing proteins including, but not limited to, collagen, elastin, gelatin, and chitosan. Genipin may be prepared by oxidation followed by reduction and hydrolysis or by enzymatic hydrolysis of the parent compound geniposide. Alternatively, racemic genipin may be prepared synthetically.

Genipin is a natural crosslinker for proteins, collagen, gelatin, and chitosan. Genipin, a bifunctional dimer, may react with itself, additional components of the compositions of the present disclosure, and/or tissue, for example endogenous collagen, to crosslink, i.e., bind, the composition of the present disclosure to tissue.

Genipin, its derivatives, analogs, any stereoisomers, or mixtures of stereoisomers of genipin, or any combination thereof, is as the reactive component. In embodiments, the genipin may possess multiple reactive groups. For example, a first reactive group on the genipin can be used to chemically bond with the other components described above to form a composition of the present disclosure, and a second reactive group on the genipin can be used to chemically bond the resulting composition to tissue. Chemical bonding refers to all types of chemical bonding, including covalent bonding, crosslinking, ionic bonding, and the like.

While genipin itself is colorless, it may react spontaneously with amine containing substituents to form blue pigments. Surprisingly, it has been found that compositions of the present disclosure including the polysaccharide and salts described above do not form a blue color when reacted in vitro but, instead, are milky white, with no change in color over time. However, upon introduction in vivo, the genipin may react with endogenous tissue to form a blue color, at least at the point of attachment of a composition of the present disclosure with tissue.

In embodiments, the reactive component may be combined with the other components described above as a solution. Suitable solvents for use in forming such a solution include, but are not limited to, water; saline; buffer salts; alcohols including methanol, ethanol and propanol; dimethyl sulfoxide; dimethylformamide; chlorinated hydrocarbons (such as methylene chloride, chloroform, 1, 2-dichloro-ethane); acetone; and aliphatic hydrocarbons such as hexane, heptene, and ethyl acetate, as well as combinations of the foregoing. Methods for forming such solutions are within the purview of those skilled in the art and include, but are not limited to, mixing, blending, sonication, heating, combinations thereof, and the like.

The reactive component may be present in such a solution in an amount from about 0.001 % by weight of the solution to about 10% by weight of the solution, in embodiments from about 0.1 % by weight of the solution to about 5% by weight of the solution, in some embodiments about 0.5% by weight of the solution.

At least one of the components, i.e., the polysaccharide, the salt, or the reactive components, should be in solution, with the other components, optionally in solution, added thereto. For example, as noted above, the polysaccharide may be in a solution. The reactive component may also be in a solution, or combined with the polysaccharide solution. The salt may be in a solution or combined with a polysaccharide solution, a reactive component solution, or both. As noted above, in some embodiments one, two, or all three of the components may be in a separate solution. The components may be combined in any order, or all at once, to form a composition of the present disclosure. The solutions may be mixed, stirred, shaken, combinations thereof, and the like, to enhance mixing and formation of a composition of the present disclosure.

Sodium alginate combined with calcium carbonate and genipin to form a composition of the present disclosure.

The polysaccharide may thus be present in a composition of the present disclosure in an amount of from about 0.1% by weight to about 10% by weight of the composition, in embodiments from about 0.5% by weight to about 5% by weight of the composition.

The salt may be present in a composition of the present disclosure in an amount of from about 0.1 % by weight to about 10% by weight of the composition, in embodiments from about 0.5% by weight to about 5% by weight of the composition.

The reactive component, in embodiments genipin, may thus be present in an amount of from about 0.001% by weight to about 10% by weight of a composition of the present disclosure, in embodiments from about 0.1 % weight to about 5% weight of a composition of the present disclosure.

Tissue contains a variety of extracellular matrix materials, including collagen, elastin, glycosaminoglycans, and other proteins. During introduction of the above components, the reactive component may not only assist in forming the gel of the present disclosure, but may also bond with the surrounding tissue, including proteins of the surrounding tissue, thereby affixing the gel to tissue.

The amount of time necessary for the reactive component to bind to tissue may vary from about 5 seconds to about 24 hours, in embodiments about 60 minutes to about 12 hours. The amount of time may vary depending upon the concentration of reactive component, the other components utilized in the composition of the present disclosure, the pH of the mixture, combinations thereof, and the like.

The above three components, i.e., the polysaccharide, salt, and reactive component, some or all of which may be in solution, may be introduced in vivo utilizing any method within the purview of those skilled in the art. Such methods include, but are not limited to, mixing, blending, dripping, brushing, and the like, or any other direct manipulation of the components on a tissue surface, or spraying of the components onto the surface of tissue. In open surgery, application by hand, forceps or the like is contemplated. In endoscopic surgery, the compositions can be delivered through the cannula of a trocar, and spread at the site by any device within the purview of those skilled in the art.

For example, in some embodiments the polysaccharide, salt, and reactive component may be in a single or multiple solutions, which may then be combined using mixing with a simple device such as a spatula. In other embodiments, the components in solution may be combined by simply placing the components into a first syringe and expelling the contents of the first syringe into a second syringe, followed by expelling the contents of the second syringe into the first syringe, and repeating this process between the two syringes until the components are mixed.

In embodiments, the components, optionally in a single or multiple solutions, may be combined prior to administration. In other embodiments, the components, optionally in a single or multiple solutions, may be combined at the time of administration. One example includes keeping each of the components separate from each other, and spraying the individual ingredients in a consecutive manner onto the same location, thereby allowing the ingredients to mix and form a gel in situ. In other embodiments, two of the components may be kept together, but separate from the third component, with a gel formed upon combining the three components.

Another example includes keeping the polysaccharide, salt, and reactive component separate from each other, and spraying the three ingredients simultaneously through the same device such as a sprayer or nozzle, thereby allowing the ingredients to mix while being sprayed onto tissue, at which time they will form a gel in situ. Methods for combining the three components at the time of administration are within the purview of those skilled in the art and include, for example, dispensing the three components from a conventional adhesive dispenser, which typically provides mixing of the components prior to the dispenser. Such dispensers are disclosed, for example, in U.S. Patent Nos. 4,978,336; 4,361,055; 4,979,942; 4,359,049; 4,874,368; 5,368,563; and 6,527,749.

In other embodiments, the three components may be combined prior to administration in vivo, thereby permitting some cross-linking to occur prior to administration. In such an embodiment, the composition of the present disclosure may then be applied in vivo and manipulated to fill a desired shape and volume before the composition has formed a gel; once the composition has formed a gel in vivo, it should closely match the shape and volume of the space to which it has been applied and previously manipulated to fill.

Upon mixing, the polysaccharide, salt, and reactive component form a flowable liquid having low viscosity of from about 10⁻³ Pa/s to about 100 Pa/s (0.01 Poise to about 1000 Poise) in embodiments from about 10-2 Pa/s to about 50 Pa/s (0.1 Poise to about 500 Poise). After a period of time during which the composition remains in this flowable state, in embodiments at least about 2.5 hours, gellation starts to occur thereby forming a composition of the present disclosure. As noted above, this delayed gelation to form a composition of the present disclosure means the components do not gel as a composition of the present disclosure until from about 3 hours to about 5 hours after combining the components, in embodiments from about 4 hours to about 4.5 hours after combining the components. The gelling reaction may be conducted at temperatures of from about 20°C to about 40° C, in embodiments from about 25°C to about 37° C. The exact reaction conditions for achieving gelling of the compositions of the present disclosure depend upon a variety of factors, including the components utilized to form the compositions, the amounts utilized, the particular solvents, if any, and the like.

In embodiments, compositions of the present disclosure may be combined with other materials, which may also be thermoresponsive. Examples of these additional thermoresponsive materials include, but are not limited to, polyoxyalkylene block copolymers, including polyethylene oxide-polypropylene oxide copolymers known as "poloxamers" and commercially available under the trade names PLURONIC^{®} (BASF Corp.) or SYNPERONIC^{®} (ICI); chitosans; hyaluronic acid (HA) and its derivatives, including copolymers of hyaluronic acid and polyethylene glycol (HA-PEG), copolymers of hyaluronic acid and PLURONICS (HA-PLURONICS); copolymers of polyethylene glycol and polylactic acid (PEG-PLLA); n-isopropylacrylamides (NIPAMs); combinations thereof, and the like.

### Additional Implants

In yet other embodiments, the compositions of the present disclosure may be combined with a separate implant and utilized to adhere the implant to tissue. Implants which may be combined with compositions of the present disclosure include sutures, staples, stents, meshes, tapes, gauzes, soft tissue repair devices, grafts, buttresses, bands, ribbons, grafts, scaffolds, wound dressings, foams, and the like.

The implant may be made of any material that can be used in surgical procedures. The implant may be any biocompatible natural or synthetic material. It should be understood that any combination of natural, synthetic, bioabsorbable and/or non-bioabsorbable materials may be used to form the implant.

Some non-limiting examples absorbable materials from which the implant may be made include, poly(lactic acid), poly (glycolic acid), poly (hydroxybutyrate), poly (phosphazine), polycaprolactone, aliphatic polyesters, glycerols, poly(amino acids), copoly (ether-esters), polyalkylene oxalates, polyamides, poly (iminocarbonates), polyalkylene oxalates, polyoxaesters, polyorthoesters, trimethylene carbonate, caprolactone, dioxanone, glycolic acid, lactic acid, glycolide, lactide, polyphosphazenes, and copolymers, block copolymers, homopolymers, blends, and combinations thereof.

Other materials which may be utilized to form an implant include non-absorbable materials such as polyamides, aramides, expanded polytetrafluoroethylene, polyethylene terephthalate (PET), polytetrafluoroethylene (PTFE), polyurethane, polyvinylidene difluoride (PVDF), polybutester, metals, alloys such as magnesium alloys, polyolefins such as polypropylene, combinations thereof, and the like.

In embodiments, natural polymers may be used in forming the implant. Suitable polymers include, but are not limited to, collagen, gelatin, fibrin, fibrinogen, elastin, keratin, albumin, hydroxyethyl cellulose, cellulose, oxidized cellulose, hydroxypropyl cellulose, carboxyethyl cellulose, carboxymethyl cellulose, homopolymers thereof, copolymers thereof, and combinations thereof.

Other suitable polymers for forming the implant include chitosan, amino functional Dextran, polylysine, any protein or peptide containing at least one primary amine, combinations thereof, and the like.

In embodiments, the implant may be in the form of a mesh. Techniques for forming a mesh are within the purview of those skilled in the art and include, for example, casting, molding, needle-punching, hooking, weaving, rolling, pressing, bundling, braiding, spinning, piling, knitting, felting, drawing, splicing, cabling, extruding, and/or combinations thereof.

Filaments utilized to produce the strands of a mesh implant may have a diameter of from about 10 µ to about 150 µ, in embodiments from about 0.08 mm to about 0.1 mm.

The mesh thus produced may have a thickness of from about 0.2 mm to about 5 mm, in embodiments from about 1 mm to about 3 mm. The strands may be spaced apart to form pores of from about 100 microns to about 2000 microns in diameter, in embodiments, from about 200 microns to about 1500 microns, in other embodiments from about 750 microns to about 1250 microns in diameter. Examples of various meshes include those disclosed in U.S. Patent Nos. 6,596,002; 6,408,656; 7,021,086; 6,971,252; 6,695,855; 6,451,032; 6,443,964; 6,478,727; 6,391,060; and U.S. Patent Application Publication No. 2007/0032805.

In other alternate embodiments, meshes including composite meshes which have at least one substrate layer and one or more layers having a porous or non-porous construction may be used in forming a reactive implant.

In embodiments a composite mesh may have a porous layer. A porous layer has openings or pores over at least a portion of a surface thereof. The pores may be in sufficient number and size so as to interconnect on a portion of the surface of the porous layer, partially across the thickness of the porous layer, entirely across the thickness of the porous layer, or combinations thereof. Those skilled in the art reading the present disclosure will envision other pore distribution patterns and configurations for the porous layer. Suitable materials for forming the porous layer include, but are not limited to, open or closed cell foams as described above.

The porous layer may be made from any biocompatible natural or synthetic material as described above. The material from which the porous layer is formed may be bioabsorbable or non-bioabsorbable. The porous layer can take the form of foams, fibers, filaments, meshes, woven and non-woven webs, compresses, pads, powders, flakes, particles, and combinations thereof as described above. Suitable techniques for forming the porous layer are within the purview of those skilled in the art and include, lyophilization, weaving, solvent evaporation, and the like.

In other embodiments, a composite mesh may have a non-porous layer. Where present, a non-porous layer may be made from any biocompatible natural or synthetic material. The material from which the non-porous layer is formed may be bioabsorbable or non-bioabsorbable as described above. The non-porous layer may be a film or sheet. A non-porous layer may retard or prevent tissue ingrowth from surrounding tissues thereby acting as an adhesion barrier and preventing the formation of unwanted scar tissue. For example, a collagen composite mesh such as a PARIETEX^{™} mesh (from Covidien) may be used in forming a reactive implant possessing a composition of the present disclosure. The PARIETEX^{™} mesh is a 3-dimensional polyester weave with a resorbable collagen film bonded on one side.

Where the composition of the present disclosure is applied to an implant and utilized to adhere the implant to tissue, the composition of the present disclosure may be applied to an implant utilizing any method within the purview of those skilled in the art. In embodiments, a composition of the present disclosure may be applied to an implant by spraying, dip coating, submersion, deposition, any other physical contact between the implant and composition, combinations thereof, and the like.

For example, the implant may be combined with a composition of the present disclosure possessing a reactive component having at least one free reactive group capable of chemically bonding with living tissue. In certain embodiments, the reactive component may crosslink with itself, the other components of the composition of the present disclosure and around and/or throughout the implant, while maintaining free reactive groups for crosslinking with a tissue surface. In other embodiments, the reactive component can crosslink with an amine-containing implant or an amine-containing coating thereon and further react to tissue. In alternate embodiments, a first reactive group of the reactive component can be used to chemically bond to the implant and a second reactive group of the reactive component can be used to chemically bond the implant to tissue.

In some embodiments, the composition of the present disclosure can be immobilized to the implant by reacting with itself. For example, the reactive component can react with itself and/or the other components of the composition of the present disclosure, encapsulating the implant, forming an intricate network encompassing the implant, or portions thereof. In certain embodiments, the reactive component may not chemically bond to the implant (if no free amines are present). The reactive component may also maintain free reactive groups for further reacting with tissue.

In alternate embodiments, the composition of the present disclosure can be immobilized to the implant by chemical bonding with the implant. Thus, the reactive component may have more than two reactive groups. For example, a first reactive group of the reactive component may react with the implant and/or the other components of a composition of the present disclosure and a second reactive group may remain free for reacting with tissue. More than one reactive group may be free for reacting with tissue; in embodiments from about 1 reactive group to about 8 reactive groups may be free for reacting with tissue. For example, the reactive component may be reactive to a proteinaceous implant. The chemical reaction between the reactive component and the implant may bind the composition of the present disclosure to the implant while leaving some reactive groups unreacted for future chemical reactions with a tissue surface in situ.

In another embodiment, the composition of the present disclosure possessing a reactive component may be supplied as a coating on the medical device, sometimes referred to herein as a "reactive coating." Methods for coating medical devices are within the purview of those skilled in the art, including but not limited to spraying, dipping, brushing, vapor deposition, co-extrusion, capillary wicking, film casting, molding, solvent evaporation, and the like. The composition of the present disclosure may be combined with the implant in the form of a coating, film, foam, or powder on at least a portion of the implant.

The composition of the present disclosure may be present in an amount of from about 0.001% by weight to about 10% by weight of the implant, in embodiments, from about 0.05% weight to about 5% weight of the implant.

Alternatively, the composition of the present disclosure may be immobilized to the implant through mechanical interactions such as wicking into pores or capillary action. For example, with woven or knitted implants such as grafts or meshes, a solution including the composition of the present disclosure may be physically entrapped in pores or between fibers. The implant may be further dried at a specified temperature and humidity level, removing residual solvent and leaving behind a reactive coating, creating a reactive implant.

Alternatively, the reactive coating may be applied to the device prior to implantation, for example soaking the medical device in the operating room, prior to implantation.

For example, a composition of the present disclosure may be supplied in a conduit to be used with a specialized injectable package material containing an implant. Examples of an injectable package are disclosed in U.S. Patent Application Publication No. 2007/0170080 filed January 26, 2006. The composition of the present disclosure may be injected into the implant package any time prior to surgical use. In embodiments, the composition of the present disclosure, water soluble or dispersible, saturates and swells the implant in preparation for use. A bioactive agent may also be added either to the composition of the present disclosure or directly into the implant package at the time of use.

In other embodiments, the composition of the present disclosure may be contacted with an implant by flooding the implant, for example a mesh, with the reactive component so that an intricate network is formed around the implant and/or through the implant or portions thereof, for example the pores of a mesh, optionally bonding with the implant. The free reactive groups may then bond to tissue, thereby affixing the implant to tissue.

Upon reacting with amine-containing tissues, the reactive implant should fixate to tissue within a useful time range. In alternate embodiments, the reactive component may be chemically "shielded" or "blocked" in aid of slowing the reaction with tissue, or the reactive component may simply have slow reaction kinetics.

The amount of time necessary for the reactive component of the composition of the present disclosure to bind the implant to tissue may vary from about 1 second to about 6 hours, in embodiments about 30 seconds to about 60 minutes.

In embodiments, the composition of the present disclosure can be immobilized to the implant by chemical bonding. For example, a free reactive group of genipin from the composition may react with the implant and a second free reactive group from the genipin may react with tissue. For example, the composition may react with functional groups in tissue such as primary amino groups, secondary amino groups, hydroxyl groups, combinations thereof, and the like thereby fixing the implant in place.

### Optional Bioactive Agents

A variety of optional ingredients may also be added to the compositions of the present disclosure including, but not limited to, surfactants, antimicrobial agents, colorants, preservatives, imaging agents, e.g., iodine or barium sulfate, or fluorine, or medicinal agents. In some embodiments, the present compositions may optionally contain one or more bioactive agents.

The term "bioactive agent", as used herein, is used in its broadest sense and includes any substance or mixture of substances that have clinical use. Consequently, bioactive agents may or may not have pharmacological activity per se, e.g., a dye. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be added to a composition of the present disclosure in any suitable form of matter, including powders, particulates, liquids, gels, combinations thereof, and the like.

Examples of classes of bioactive agents which may be utilized in accordance with the present disclosure include anti-adhesives, antimicrobials, anti-infectives, anti-thrombotics, analgesics, antipyretics, anesthetics, antiepileptics, antihistamines, antiinflammatories, anti-proliferatives, cardiovascular drugs, diagnostic agents, chemotherapeutic agents, telomerase inhibitors, polymer drugs including polyaspirin and polydiflunisal, anti-platelet drugs, platelet activating drugs, angiogenic agents, gene therapy agents, protein therapeutics, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Suitable antimicrobial agents include those agents which by themselves or through assisting the body can help destroy or resist microorganisms which may be pathogenic. Examples of antimicrobial agents include antibiotics; quorum sensing blockers; surfactants; metal ions; antiseptics; disinfectants; anti-virals; anti-fungals; triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides, such as tobramycin and gentamicin; rifampicin; bacitracin; neomycin; chloramphenicol, miconazole; quinolones such as oxolinic acid, norfloxacin, nalidixic acid, pefloxacin, enoxacin and ciprofloxacin; penicillins such as oxacillin and pipracil; nonoxynol 9; fusidic acid; cephalosporins, and combinations thereof. In addition, antimicrobial proteins and peptides such as bovine lactoferrin and lactoferricin B, and antimicrobial polysaccharides such as fucans and derivatives thereof, may be included as a bioactive agent in a composition of the present disclosure.

Other bioactive agents which may be included as a bioactive agent in a composition of the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be added to a composition of the present disclosure include viruses and cells; peptides, polypeptides and proteins, analogs, muteins, and active fragments thereof, such as immunoglobulins, antibodies, nanobodies, cytokines (e.g., lymphokines, monokines, chemokines); blood clotting factors; hemopoietic factors; interleukins (IL-2, IL-3, IL-4, IL-6); interferons ((3-IFN, (a-IFN and y-IFN); erythropoietin; nucleases; tumor necrosis factor; colony stimulating factors (e.g., GCSF, GM-CSF, MCSF); insulin; anti-tumor agents and tumor suppressors; blood proteins; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA, DNA intercalators, RNA; oligonucleotides; polynucleotides; and ribozymes.

Naturally occurring polymers, including proteins such as collagen and derivatives of various naturally occurring polysaccharides such as glycosaminoglycans, can optionally be incorporated into the compositions of the present disclosure as a bioactive agent.

A single bioactive agent may be utilized in the present compositions or, in alternate embodiments, any combination of bioactive agents may be utilized in the present compositions.

Various advantages may be had from utilizing a gel of the present disclsoure. For example, the delayed gelation exhibited by a composition of the present disclosure may be advantageous in certain medical procedures, where the doctor and/or surgeon introducing the composition needs time to introduce the comoposition as a low viscosity liquid, and then shape the tissue and/or redistribute the composition prior to gelation in a permanent form. Thus, the compositions of the present disclosure may be useful in many surgical procedures where a slower gelation time is beneficial including, but not limited to, sphincter augmentation, including lower esophageal sphincter bulking to treat gastroesophageal reflux disease (GERD), periurethral bulking to treat urinary incontinence, creating cushions between tissue layers to assist in tissue dissections and/or resections, for example in polypectomy procedures, preventing adhesions, plastic surgery as a dermal filler, combinations thereof, and the like.

For example, compositions of the present disclosure may be utilized in sphincter augmentation applications including, but not limited to, urinary (urethral), anal, and esophageal sphincter augmentation. Any method within the purview of those skilled in the art may be utilized to introduce a compositions of the present disclosure into a sphincter. As would be apparent to one skilled in the art, the method selected may depend, in part, upon the location of the sphincter within the body.

For example, a composition of the present disclosure may be delivered to a target tissue site to augment a mammalian sphincter, such as the lower esophageal sphincter (LES). In embodiments, a catheter assembly may be utilized to introduce the compositions of the present disclosure. Such catheter assemblies may include a flexible catheter having a distal end affixed to an injection needle may be utilized to introduce a composition of the present disclosure into the sphincter. The catheter may be coupled to a syringe at its proximal end. The syringe may contain the compositions of the present disclosure by a standard luer connection. The needle may pierce tissue at or adjacent the sphincter to deliver a composition of the present disclosure to a portion of the sphincter. Pressure may then be applied to the syringe plunger, which then injects the composition of the present disclosure into the lumen of the catheter and, subsequently, the needle.

In embodiments, compositions of the present disclosure may also be utilized in cosmetic surgery. For example, bulking of skin tissues, including fascia, subcutaneous and dermal tissues, may be used to treat skin disorders including scars, wrinkles, skin laxness, and skin thinning, and may be used in some types of cosmetic and reconstructive plastic surgery. Such disorders of the skin often are exhibited as contour deficiencies, which may be treated using the compositions of the present disclosure. Contour deficiencies in the skin can occur as a result of factors such as aging, environmental exposures, weight loss, childbearing, surgery or disease. Contour deficiencies include frown lines, worry lines, wrinkles, crow's feet, marionette lines, stretch marks, internal and external scars, combinations thereof, and the like. Augmentation of the skin layers with compositions of the present disclosure may thus reduce or eliminate such contour deficiencies.

The compositions may be introduced into the desired skin layer, in embodiments by injection, without having to worry about over-swelling which may distend tissue. As a wrinkle filler, the compositions of the present disclosure can be injected or otherwise placed subcutaneously in a liquid form, with gelling occurring after administration. The compositions of the present disclosure can advantageously be shaped or spread thinly to achieve the desired effect while still in a liquid form.

For cosmetic or reconstructive surgery applications, compositions of the present disclosure can be applied to a selected area of the body in a liquid form (or can be formed prior to insertion as described herein), and can be manipulated into the desired shape or to fill a desired volume prior to solidification. Reconstructive surgery or aesthetic enhancement may incorporate the compositions of the present disclosure. Regions of the face, such as cheeks, nose, ears, and skin adjacent the eyes (soft tissue) can be reconstructively augmented or enhanced using the compositions of the present disclosure.

While current cosmetic bulking products, many of which are based upon natural materials such as crosslinked collagen, gelatin, hyaluronic acid, and the like, may be absorbed by the body, which limits their tissue persistence and may require overcorrection and repeat injections, the synthetic gels of the present disclosure are not absorbed, or at least not to the same extent as a natural material, which may avoid the need for overcorrection and repeated injections. Alternatively, the synthetic gels of the present disclosure can be formulated to degrade upon demand and be absorbed by the body by including degradable linkages susceptible to selective cleavage by the introduction of a reactive agent, such as amide or ester linkages cleavable by enzymatic or non-enzymatic means. The delay of gelation may also permit manipulation of the gel in vivo prior to solidification, which may be especially useful in cosmetic applications as it permits adjustment of the size and/or shape of the gel in vivo to maximize placement and shape of the composition for correcting cosmetic defects.

Moreover, gels of the present disclosure may be used for applications such as scaffolds for orthopedic applications, tissue engineering, cell-seeding scaffolds, matrices for drug delivery, combinations thereof, and the like. Where the composition of the present disclosure is intended for delivery of a drug or protein, the amounts of the components can be adjusted to promote the initial retention of the drug or protein in the gel and its subsequent release. Methods and means for making such adjustments will be readily apparent to those skilled in the art.

For example, in some embodiments, the gels of the present disclosure may be utilized to deliver cells in vivo. For example, living cells such as chondrocytes may be added to a gel of the present disclosure. The gel can be a flowing liquid capable of carrying the cells into the body to a desired location, whereupon the gel becomes solid and the cells remain viable and active. In the case of chondrocytes, the formation of cartilage may thus be accomplished with a gel of the present dislcosure. For example, the chondrocyte-seeded composition of the present disclosure can be in a liquid form easily injected during a minimally invasive surgical procedure to a focal cartilage lesion or void. The liquid will fill and adhere to the cartilage lesion or void, and then become a solid, where it can act as a three-dimensional scaffold for the cells.

The compositions described herein can also be suitable for use with delicate tissues where sutures, clamps or other conventional tissue closure mechanisms may cause further tissue damage. For example, the compositions of the present disclosure may be used to seal or adhere delicate tissue together, such as lung tissue, in place of conventional tools that may cause mechanical stress. The present compositions can also be used to seal air and/or fluid leaks in tissue as well as to prevent post-surgical adhesions and to fill voids and/or defects in tissue.

The compositions described herein can also be used as sealants. When used as a sealant, a composition of the present disclosure can be used in surgery to prevent or inhibit bleeding or fluid leakage after a surgical procedure. It can also be applied to prevent air leaks after pulmonary surgery. Compositions herein may be applied directly to the desired area in at least an amount sufficient to seal off any defect in the tissue and seal off any fluid or air movement.

The present compositions can also be used to prevent post surgical adhesions. In such an application, a composition of the present disclosure may be applied and allowed to gel to form a layer on surfaces of internal tissues in order to prevent the formation of adhesions at a surgical site during the healing process.

A gel of the present disclosure includes sodium alginate in combination with calcium carbonate and genipin. The mixture can gel on its own, with the gel further crosslinking with surrounding tissue due to the presence of genipin. In other embodiments, a two-component system may be utilized, with alginate, calcium carbonate, and genipin combined as the first component; and a protein solution, such as gelatin, albumin, blood plasma, or an amine containing solution such as chitosan, combinations thereof, and the like, utilized as the second component. Upon mixing the two components, cross-linking will occur thereby forming a solid gel.

By varying the selection of the compounds utilized to form the bioadhesive composition, the strength and elasticity of the bioadhesive composition can be controlled, as can the gelation time.

The following Examples are being submitted to illustrate embodiments of the present disclosure. These Examples are intended to be illustrative only and are not intended to limit the scope of the present disclosure. Also, parts and percentages are by weight unless otherwise indicated.

### EXAMPLE 1

A composition of the present disclosure was prepared as follows. A solution of sodium alginate in water, having a low viscosity of about 0,01 Pa/s (0.1 Poise). a pH of about 7, and a concentration of about 1%, was combined with a calcium carbonate solution having a concentration of about 0.5% by weight in water and a pH of from about 8 to about 9, and a genipin solution at a concentration of about 0.5% by weight in water and a pH of about 7.5. Upon mixing, the material remained flowable with a viscosity of about 0,02 Pa/s (0.2 Poise) at room temperature. The material was left overnight, about 15 hours, after which time the material was a solid white gel that did not flow or break down, even when subjected to vigorous shaking.

An additional composition was prepared as described and examined to determine the time for gelation. It was found that, upon mixing of the above 3 components, the liquid mixture had a low viscosity of about 0.2 Poise at room temperature, which stayed constant for about 2.5 hours. Soon after that time, the composition's storage modulus (G ' (in dyne/cm²)) and viscosity (Eta* (in Poise)) started to sharply increase. The results are depicted in the Figure. The liquid turned into a non-flowing gel between about 3 hours and about 4 hours, as determined by visual inspection and a tilt test. The crossover gel point, where G '=G" (G" is the loss modulus in dyne/cm²) was obtained from an ARES rheometer (from TA Instruments), utilizing a time-sweep run, with the rheometer operated at a constant strain of about 50%, a constant frequency of about 10 rad/seconds, and a constant temperature of about 25°C. The crossover gel point was determined to occur at a time of from about 4 hours to about 5 hours after mixing the three components, as depicted in the Figure. The formed gel had a firm texture, with a storage modulus (G ') of about 1000 dyne/cm² and a white color.

### COMPARATIVE EXAMPLE 1

Various combinations of the solutions described in Example 1 were made to determine gelling characteristics. A solution of 0.5% by weight of genipin in water was prepared with a pH of from about 7.5 to about 8. A solution of 0.5% by weight calcium carbonate was added thereto to see if the combination of genipin and calcium carbonate would gel similar to Example 1. No gelling was observed.

Similarly, an alginate solution was prepared at a concentration of about 1% by weight by dissolving sodium alginate in deionized water. The resulting alginate solution had a pH of about 9. This alginate solution was combined with the above genipin solution (no calcium carbonate) to see if the combination of genipin and alginate would gel similar to Example 1. No gelling was observed.

Similarly, an alginate and calcium carbonate solution was prepared by combining the about 1% alginate solution described above with about 0.5% CaCO₃ at a pH greater than about 6. No gellation was observed, even after a few months.

As noted above, compositions of the present disclosure exhibit delayed gelation, of from about 3 hours to 5 hours, in embodiments from about 4 hours to about 4.5 hours. Interestingly, the data demonstrates that all 3 components may be necessary to obtain the delayed gelation: mixing genipin with an alginate solution, but without calcium carbonate, at any pH of from about 4 to about 10, did not produce a gel; similarly, combining genipin with calcium carbonate, but without alginate, did not produce a gel; and combining alginate with calcium carbonate, but without genipin, at a pH greater than about 6 did not produce gelation. As the pH of the alginate-genipin solution is greater than about 6, in embodiments from about 6 to about 8.5, in embodiments from about 7 to about 8, in some embodiments about 7.5, the pH may not be low enough for the calcium carbonate to solubilize/ionize to crosslink the alginate. Generally, the pH of the solution would have to be below about 5 for calcium to solubilize/ionize and crosslink the alginate, and at that point one would observe instant gelation, not the delayed gelation observed where all 3 of the above components are utilized to form a composition of the present disclosure.

### EXAMPLE 2

The gel of Example 1, made with a solution of 1% by weight sodium alginate, about 0.5% by weight CacO₃, and about 0.5% by weight genipin, was prepared as described in Example 1 and allowed to sit at room temperature, from about 20°C to about 25°C, for about 48 hours.

After about 48 hours, a 25% by weight gelatin solution was prepared by dissolving about 25 grams of gelatin in about 75 grams of deionized water. About 10 mL of the resulting gelatin solution was added to the gel of Example 1 and allowed to sit overnight, from about 16 hours to about 24 hours.

Upon examination the following day, the white gel from Example 1 had turned dark blue in the presence of the gelatin and had become much more stiff as determined by visual inspection and manual manipulation. While not wishing to be bound by any theory, it appears that at least a portion of the genipin utilized to form the gel of Example 1 remained active and capable of reacting with the amino groups of the gelatin, thereby forming the dark blue color.

The speed with which the reaction with gelatin occurred, as evidenced by the dark blue color and speed of reaction, may have been catalyzed by the polysaccharide (alginate) or salt (CaCO₃) of the gel of Example 1, as the reaction between the gel of Example 1 and gelatin occurred more rapidly than the reaction of gelatin with genipin alone.

### EXAMPLE 3

A solution was prepared of about 1% by weight of sodium alginate in water. A light powder of about 0.5% by weight CaCO₃, and about 0.5% by weight genipin, was added thereto. A flowing milky white suspension was formed, having a viscosity of about 0.2 Poise.

Two rectangular pieces were cut from a mesh made of polylactic acid (from Covidien), each piece being from about 1 cm X about 2 cm. The first mesh piece was dip coated for about 10 seconds in the flowing milky suspension, and left to gel in a closed Petri dish at room temperature, from about 20°C to about 25°C, for about 4 hours. It was observed that the milky white suspension had turned into a white solid gel on and within the pores of the first mesh piece.

The second mesh piece was fully submerged in the milky suspension. A cover was placed over the container containing the milky suspension and the second mesh piece and left to gel at room temperature, from about 20°C to about 25°C. After about 4 hours, the flowing suspension turned into a non-flowing white gel. The second mesh piece was removed from the gel, sometimes referred to herein as a gel mold, and placed in an empty dish. The gel mold was placed in a separate empty dish.

A gelatin solution having about 25% by weight gelatin was poured onto the first mesh piece, the second mesh piece, and the gel mold. The gelatin was utilized to simulate tissue. The three dishes, one with the first mesh piece, the second with the second mesh piece, and the third with the gel mold, were placed in an oven at a temperature of about 37°C and left overnight, from about 12 hours to about 24 hours.

The dishes were removed from the oven, at which time a dark blue color was noted in all three dishes, suggesting that the genipin had crosslinked with the amine groups found in the gelatin. Attempts to remove the pieces of mesh from their dishes demonstrated that the meshes were well stuck and encapsulated by the gelatin solution. Thus, it appears that the genipin played an important role in both the initial formation of the milky white suspension and subsequent white gel, as well as crosslinking the gel with tissue. The above clearly demonstrates the feasibility of using a composition of the present disclosure as a reactive surgical implant

It will be understood that various modifications may be made to the embodiments disclosed herein. Therefore, the above description should not be construed as limiting, but merely as an exemplification of preferred embodiments. Those skilled in the art will envision other modifications within the scope of the present disclosure. Such modifications and variations are intended to come within the scope of the following claims.

## Claims

1. A composition comprising:
at least one polysaccharide, optionally in solution;
at least one salt, optionally in solution; and
at least one reactive component, optionally in solution,
wherein at least one of the polysaccharide, salt and reactive component are in a solution, and wherein the composition forms a gel from about 3 hours to about 5 hours after the at least one polysaccharide, at least one salt, and at least one reactive component have been combined; and **characterised in that** the at least one polysaccharide comprises sodium alginate, the at least one salt comprises calcium carbonate, and the at least one reactive component comprises genipin and wherein the pH of the composition is not below 5

2. The composition of claim 1, wherein the at least one polysaccharide is in a solution at a concentration of from about 0.1% by weight to about 10% by weight.

3. The composition of claim 1 or 2, wherein the at least one salt is in a solution at a concentration of from about 0.1 % by weight to about 10% by weight.

4. The composition of any proceeding claim, wherein the at least one reactive component is in a solution at a concentration of from about 0.001 % by weight to about 10% by weight.

5. The composition of any preceding claim, further comprising at least one bioactive agent selected from the group consisting of anti-adhesives, antimicrobials, anti-infectives, anti-thrombotics, analgesics, antipyretics, anesthetics, antiepileptics, antihistamines, antiinflammatories, anti-proliferatives, cardiovascular drugs, diagnostic agents, chemo agents, telomerase inhibitors, polymer drugs, anti-platelet drugs, platelet activating drugs, angiogenic agents, gene therapy agents, protein therapeutics, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, enzymes, and combinations thereof.

6. The composition of Claims 1 to 5, for use in sphincter augmentation, including lower esophageal sphincter bulking in the treatment of gastroesophageal reflux disease or periurethral bulking in the treatment of urinary incontinence.

## Patentansprüche

1. Zusammensetzung, umfassend:
mindestens ein Polysaccharid, gegebenenfalls in Lösung;
mindestens ein Salz, gegebenenfalls in Lösung;
mindestens eine reaktive Komponente, gegebenenfalls in Lösung;
wobei mindestens eines aus dem Polysaccharid, dem Salz und der reaktiven Komponente in Lösung ist und wobei die Zusammensetzung von ungefähr 3 Stunden bis ungefähr 5 Stunden, nachdem das mindestens eine Polysaccharid, das mindestens eine Salz und die mindestens eine reaktive Komponente miteinander gemischt worden sind, ein Gel bildet und **dadurch gekennzeichnet ist, dass** das mindestens eine Polysaccharid Natriumalginat umfasst, das mindestens eine Salz Calciumcarbonat umfasst und die mindestens eine reaktive Komponente Genipin umfasst und wobei der pH-Wert der Zusammensetzung nicht unter pH 5 ist.

2. Zusammensetzung nach Anspruch 1, wobei das mindestens eine Polysaccharid mit einer Konzentration von ungefähr 0,1 Gew.-% bis ungefähr 10 Gew.-% in einer Lösung ist.

3. Zusammensetzung nach Anspruch 1 oder 2, wobei das mindestens eine Salz mit einer Konzentration von ungefähr 0,1 Gew.-% bis ungefähr 10 Gew.-% in einer Lösung ist.

4. Zusammensetzung nach Anspruch 1, wobei die mindestens eine reaktive Komponente mit einer Konzentration von ungefähr 0,001 Gew.-% bis ungefähr 10 Gew.-% in einer Lösung ist.

5. Zusammensetzung nach einem der vorhergehenden Ansprüche, ferner umfassend mindestens ein bioaktives Mittel, das ausgewählt ist aus der Gruppe, bestehend aus Antiklebmitteln, antimikrobiellen Mitteln, Anti-Infektionsmittel, Antithrombosemitteln, schmerzstillenden Mitteln, fiebersenkenden Mitteln, Anästhetika, Antiepileptika, Antihistaminika, entzündungshemmenden Mitteln, antiproliferativen Mitteln, kardiovaskulären Arzneimitteln, diagnostischen Mitteln, chemotherapeutischen Mitteln, Telomerase-Inhibitoren, Polymer-Arzneimittel, Thrombozytenaggregationshemmern, Thrombozytenaktivierenden Arzneimitteln, angiogenen Mitteln, gentherapeutischen Mitteln, Proteintherapeutika, Sympathomimetika, Cholinomimetika, Antimuskarinika, Antispasmodika, Hormonen, Wachstumsfaktoren, Muskelrelaxanzien, Blockern adrenerger Neurone, antineoplastischen Mitteln, immunogenen Mitteln, Immunsuppressiva, gastrointestinalen Arzneimitteln, Diuretika, Steroiden, Lipiden, Lipopolysacchariden, Polysacchariden, Enzymen und Kombinationen davon.

6. Zusammensetzung nach Anspruch 1 bis 5 zur Verwendung in der Schließmuskel-Vergrößerung, einschließlich des Injizierens von Bulking Agents in den Schließmuskel der Speiseröhre bei der Behandlung der gastroösophagealen Refluxkrankheit oder dem periurethralen Injizieren von Bulking Agents bei der Behandlung der Harninkontinenz.

## Revendications

1. Composition comprenant :
au moins un polysaccharide, de manière facultative en solution ;
au moins un sel, de manière facultative en solution ; et
au moins un composant réactif, de manière facultative en solution,
dans lequel au moins un du polysaccharide, du sel et du composant réactif est dans une solution, et dans lequel la composition forme un gel environ 3 heures à environ 5 heures après que l'au moins un polysaccharide, l'au moins un sel et l'au moins un composant réactif ont été combinés ; et **caractérisée en ce que** l'au moins un polysaccharide comprend de l'alginate de sodium, l'au moins un sel comprend du carbonate de calcium et l'au moins un composant réactif comprend de la génipine et dans laquelle le pH de la composition n'est pas inférieur à 5

2. Composition selon la revendication 1, dans laquelle l'au moins un polysaccharide est dans une solution à une concentration d'environ 0,1 % en poids à environ 10 % en poids.

3. Composition selon la revendication 1 ou 2, dans laquelle l'au moins un sel est dans une solution à une concentration d'environ 0,1 % en poids à environ 10 % en poids.

4. Composition selon l'une quelconque des revendications précédentes, dans laquelle l'au moins un composant réactif est dans une solution à une concentration allant d'environ 0,001 % en poids à environ 10 % en poids.

5. Composition selon l'une quelconque des revendications précédentes, comprenant en outre au moins un agent bioactif sélectionné à partir du groupe constitué par des anti-adhésifs, des produits antimicrobiens, des anti-infectieux, des antithrombotiques, des analgésiques, des antipyrétiques, des anesthésiques, des antiépileptiques, des antihistaminiques, des anti-inflammatoires, des anti-proliférants, des médicaments cardiovasculaires, des agents de diagnostic, des agents chimio, des inhibiteurs de télomérase, des médicaments polymères, des médicaments anti-plaquette-sanguine, des médicaments activant les plaquettes sanguines, des agents angiogéniques, des agents de thérapie génique, des agents thérapeutiques protéiques, des sympathico-mimétiques, des cholinomimétiques, des antimuscariniques, des spasmolytiques, des hormones, des facteurs de croissance, des tranquillisants musculaires, des inhibiteurs neuronaux adrénergiques, des anticancéreux, des agents immunogènes, des immunosuppresseurs, des médicaments gastro-intestinaux, des diurétiques, des stéroïdes, des lipides, des lipopolysaccharides, des polysaccharides, des enzymes et des combinaisons de ceux-ci.

6. Composition selon les revendications 1 à 5, à utiliser dans l'augmentation de sphincter, y compris le gonflement de sphincter oesophagien inférieur dans le traitement de la maladie du reflux gastro-oesophagien ou le gonflement péri-urétral dans le traitement de l'incontinence urinaire.
